# EUROPEAN PATENT APPLICATION

(11) **EP 4 717 170 A1**
(43) Date of publication of application: **01.04.2026**
(21) Application number: 24849539.2
(22) Date of filing: 29.07.2024
(51) Int. Cl.: A61B 5/1455, A61B 5/00, A61B 5/145, G01J 3/10, G01J 3/42, G01N 21/25, G01N 21/31, A61B 5/024, G02B 6/12, G02F 1/01, H01S 5/02325, H01S 5/0239, H01S 5/026, H01S 5/028, H01S 5/0683, H01S 5/10, H01S 5/14, H01S 5/40, H01S 5/50

(54) **ELECTRONIC DEVICE AND WEARABLE DEVICE**

(30) Priority: 01.08.2023 KR 20230100511; 29.11.2023 KR 20230169291
(71) Applicant: Samsung Electronics Co., Ltd., Suwon-si, Gyeonggi-do 16677 (KR)
(72) Inventor: LEE, Juneyoung, Suwon-si, Gyeonggi-do 16677 (KR); KIM, Younghyun, Suwon-si, Gyeonggi-do 16677 (KR); LEE, Jeahyuck, Suwon-si, Gyeonggi-do 16677 (KR); PARK, Minho, Suwon-si, Gyeonggi-do 16677 (KR); AHN, Joongwoo, Suwon-si, Gyeonggi-do 16677 (KR)
(74) Representative: Appleyard Lees IP LLP
(86) International application number: PCT/KR2024/011021
(87) International publication number: WO 2025/028971

(57) **Abstract**

The electronic device of the disclosure may include a semiconductor optical amplifier configured to divide an outputtable wavelength band and output multiple laser lights having a designated wavelength band and designated power, a fixed array distributed Bragg reflector (DBR) grating configured to modify a wavelength of the multiple laser lights and output multiple laser lights having a modified wavelength, and an output coupler configured to allow the modulated multiple laser lights to adjust an output direction and/or angle and output the modulated multiple laser lights to an outside of the electronic device.

## Description

### [Technical Field]

The disclosure relates to an electronic device and a wearable device.

### [Background Art]

Recently, electronic devices including sensors capable of measuring users' biometric information and/or fitness data have been developed.

The electronic devices may typically be carried in a pocket or hand for use while on the move, and may also have a form of being wearable on a part of the body or various structures. The electronic device may offer health and fitness information services by utilizing an ability thereof to be worn on the user's body.

The above information may be presented as related art for the purpose of assisting in understanding the disclosure. No assertion or decision is made as to whether any of the above might be applicable as prior art with regard to the disclosure.

### [Disclosure of Invention]

In order to detect blood sugar information among user's biometric information, an electronic device may include a sensor which may detect blood sugar in a noninvasive manner based on an optical sensor (e.g., a laser). The electronic device may output light from a light source of the optical sensor. The electronic device may analyze an object by obtaining a spectrum of light transmitted and/or reflected from the object. By using multiple light sources covering different wavelengths, a wavelength range targeted by the optical sensor may be configured.

Here, in case that each light source covers wavelengths of wider band, the intensity of the output light may be lower than a determined reference. Alternatively, increasing the intensity (power) of light emitted from each light source beyond a determined reference causes a problem of reducing the range of wavelengths that each light source may cover.

An aspect of the disclosure is to provide an electronic device including an optical sensor including multiple light sources, which controls power of a light source for each determined band section.

The electronic device of the disclosure may include a semiconductor optical amplifier configured to divide an outputtable wavelength band to output multiple laser lights having a designated wavelength band and designated power, a fixed array distributed Bragg reflector (DBR) grating configured to modify a wavelength of the multiple laser lights and output multiple laser lights having a modified wavelength, and an output coupler configured to allow the modulated multiple laser lights to adjust an output direction and/or angle and output the modulated multiple laser lights to the outside of the electronic device.

A wearable device of the disclosure may include a transmission circuit, a reception circuit, and a processor, wherein the transmission circuit may include a semiconductor optical amplifier configured to divide an outputtable wavelength band to output multiple laser lights having a designated wavelength band and designated power, a fixed array distributed Bragg reflector grating (DBR) configured to modify a wavelength of the multiple laser lights and output multiple laser lights having a modified wavelength, and an output coupler configured to allow the modulated multiple laser lights to adjust an output direction and/or an angle and output the modulated multiple laser lights to the outside of the electronic device.

The electronic device and the wearable device of the disclosure may output light by controlling power of a light source for each designated band section so as to improve accuracy when measuring an object using light.

The electronic device and the wearable device of the disclosure may output light by controlling power of a light source for each designated band section so as to improve the signal-to-noise ratio when measuring an object using light.

### [Brief Description of Drawings]

With regard to the description of the drawings, the same or like reference signs may be used to designate the same or like elements.
FIG. 1 is a block view illustrating an electronic device in a network environment according to various embodiments of the disclosure.
FIG. 2A is a view illustrating an electronic device according to an embodiment of the disclosure.
FIG. 2B is a view illustrating a light output structure and a light reception structure disposed on a second surface or a rear surface of an electronic device according to an embodiment of the disclosure.
FIG. 3 is a block view illustrating a sensor circuit of an electronic device according to an embodiment of the disclosure.
FIG. 4 is a view illustrating a transmission circuit realized on a silicon-based integrated circuit.
FIG. 5 is a graph depicting density of light according to wavelength after each of multiple optical amplifier chips included in a semiconductor optical amplifier 311 irradiates water with a laser output at an identical level of power.
FIG. 6 is a graph illustrating laser power for each wavelength band output from a semiconductor optical amplifier 311 including at least five optical amplifier chips according to an embodiment of the disclosure.
FIG. 7 is a graph illustrating laser power for each wavelength band output from a semiconductor optical amplifier including at least four optical amplifier chips according to an embodiment of the disclosure.
FIG. 8 is a graph illustrating laser power for each wavelength band output from a semiconductor optical amplifier including at least three optical amplifier chips according to an embodiment of the disclosure.
FIG. 9 is a flowchart illustrating a laser output method of an electronic device according to an embodiment of the disclosure.

### [Mode for the Invention]

Hereinafter, embodiments of the disclosure will be described in detail with reference to the drawings so that those skilled in the art to which the disclosure pertains can easily implement the disclosure. However, the disclosure may be implemented in various forms and is not limited to embodiments set forth herein. With regard to the description of the drawings, the same or like reference signs may be used to designate the same or like elements. Also, in the drawings and the relevant descriptions, description of well-known functions and configurations may be omitted for the sake of clarity and brevity.

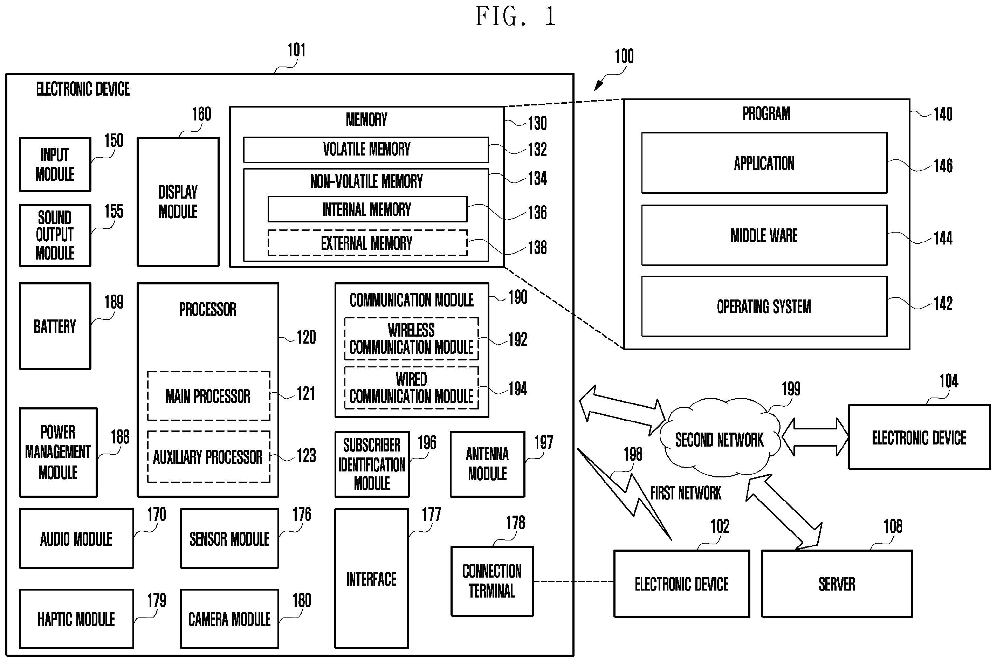

Fig. 1 is a block diagram illustrating an electronic device 101 in a network environment 100 according to various embodiments.

Referring to Fig. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may include various processing circuitry and/or multiple processors. For example, as used herein, including the claims, the term "processor" may include various processing circuitry, including at least one processor, wherein one or more of at least one processor, individually and/or collectively in a distributed manner, may be configured to perform various functions described herein. As used herein, when "a processor", "at least one processor", and "one or more processors" are described as being configured to perform numerous functions, these terms cover situations, for example and without limitation, in which one processor performs some of recited functions and another processor(s) performs other of recited functions, and also situations in which a single processor may perform all recited functions. Additionally, the at least one processor may include a combination of processors performing various of the recited /disclosed functions, e.g., in a distributed manner. At least one processor may execute program instructions to achieve or perform various functions. The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thererto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

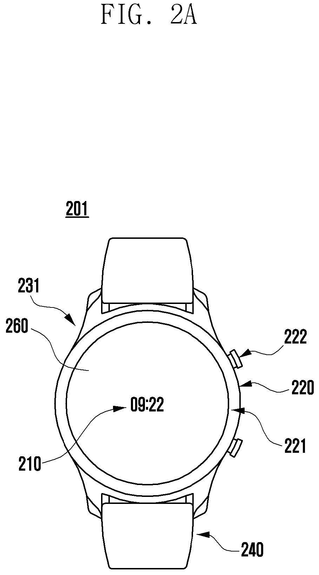

FIG. 2A is a view illustrating a front surface 231 of an electronic device 201 according to an embodiment of the disclosure.

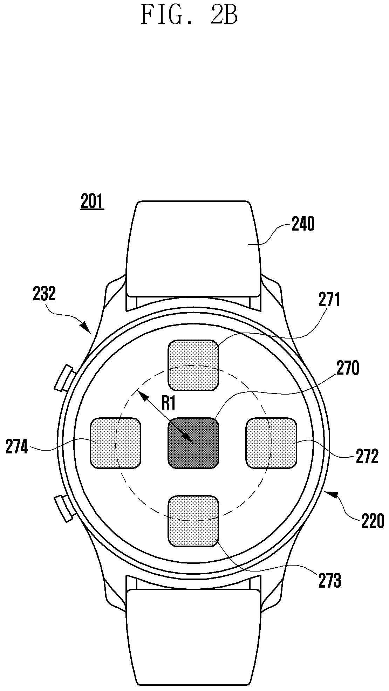

FIG. 2B is a view illustrating a rear surface 232 of the electronic device 201 according to an embodiment of the disclosure.

Referring to FIGS. 2A and 2B, the electronic device 201 in FIGS. 2A and 2B may include components similar or identical to those of the electronic device 101 in FIG. 1. However, the discloser is not limited thereto, and the electronic device 201 in FIGS. 2A and 2B may have additional or omitted components compared to the electronic device 101 in FIG. 1.

In an embodiment, the electronic device 201 may include a display 260 (e.g., the display module 160 in FIG. 1) on a front surface 231. The electronic device 201 may include a wearable device that may be worn by a user in the shape of a watch. The electronic device 201 may display an interface 210 related to a watch on at least a portion of the display 260.

In an embodiment, the electronic device 201 may include a case 220 and a band 240.

In an embodiment, the case 220 may include a bezel 221, a crown 222, and the display 260 on the outside thereof. Referring to FIG. 1, the case 220 may include, on the outside or in the inside thereof, a processor (e.g., including processing circuitry) 120, a memory 130, an input module (e.g., including input circuitry) 150, a sound output module (e.g., including sound output circuitry) 155, a display module (e.g., including a display) 160, an audio module (e.g., including audio circuitry) 170, a sensor module (e.g., including at least one sensor) 176, an interface (e.g., including interface circuitry) 177, a connection terminal 178, a haptic module (e.g., including haptic circuitry) 179, a camera module (e.g., including at least one camera) 180, a power management module (e.g., including power management circuitry) 188, a battery 189, a communication module (e.g., including communication circuitry) 190, a subscriber identity module (e.g., SIM) 196, and/or an antenna module (e.g., including at least one antenna) 197.

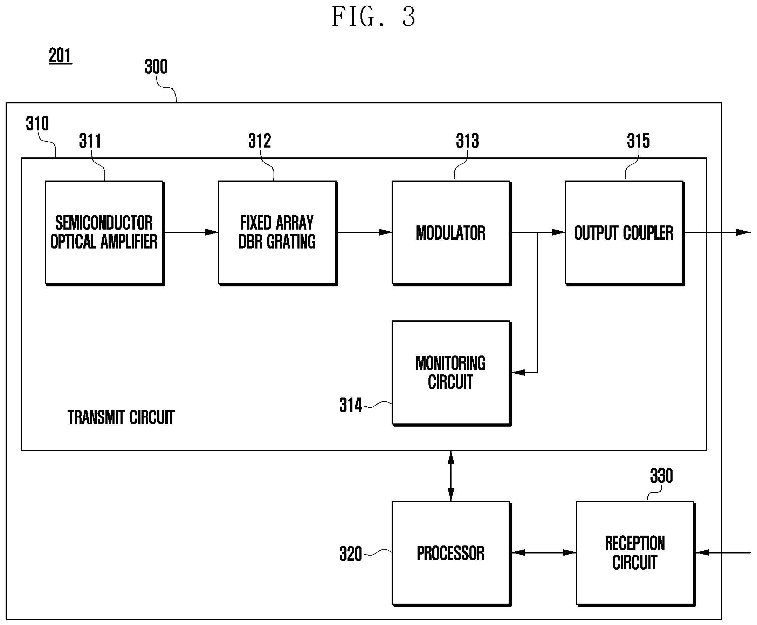

In an embodiment, the case 220 may include the display 260 disposed on the front surface 231 and at least a portion (e.g., an output coupler 315 and/or a photo detector in FIG. 3) of the sensor circuit (e.g., a sensor circuit 300 in FIG. 3) on a rear surface 232 may be disposed to the outside.

In an embodiment, at least a portion of the rear surface 232 of the case 220 may come in contact with a user through the band 240 when the electronic device 201 is worn on the user.

In an embodiment, the bezel 221 has a concentric circle shape with the circular display 260 and may have a ring shape. An inner radius of the bezel 221 may be substantially identical to that of the display 260. The bezel 221 may be disposed on an edge portion of the case 220 to protect the display 260 from external impact.

In an embodiment, the bezel 221 may rotate in at least one of clockwise or counterclockwise direction. The bezel 221 may function as an input device of the electronic device 201. When the bezel 221 rotates, the electronic device 201 may determine a rotation speed and rotation direction of the bezel 221 as a user input and control a function of the electronic device 201 according to the user input.

In an embodiment, the crown 222 may be disposed to protrude on a partial area of the case 220. The crown 222 may have a cylindrical shape. The crown 222 may connected to the case 220 based on a rotation axis and rotate. The crown 222 may correspond to a stem providing a rotation axis and may connected to the case 220 to rotate.

In an embodiment, the crown 222 may function as an input device of the electronic device 201. When the crown 222 rotates, the electronic device 201 may determine a rotation speed and rotation direction of the crown 222 as a user input and control a function of the electronic device 201 according to the user input.

In an embodiment, the band (or strap) 240 may allow the electronic device 201 to be seated on a wrist of the user. The band 240 may include various materials such as metal, rubber, and leather. The band 240 may be connected to one end of the case 220 and the band 240 connected to the case 220 may be replaceable.

In an embodiment, the electronic device 201 may include, on the rear surface 232, a light output structure 270 and multiple light reception structures 271, 272, 273, and 274. An output coupler (e.g., an output coupler 315 in FIG. 3) may be included in at least a portion of the light output structure 270. The electronic device 201 may include the multiple light reception structures 271, 272, 273, and 274 spaced a first distance R1 apart around the light output structure 270. The first distance R1 may have a designated length. Each of the multiple light reception structures 271, 272, 273, and 274 may include a reception circuit 330 and/or a photo detector.

Without limitation thereto, the electronic device 201 may include, on the rear surface 232, an optical blood flow measurement sensor (photoplethysmogram (PPG)), a heartbeat measurement sensor (electrocardiogram (ECG)), the light output structure 270, and the multiple light reception structures 271, 272, 273, and 274.

Referring to FIG. 2B, a first light reception structure 271, a second light reception structure 272, a third light reception structure 273, and a fourth light reception structure 274 may be arranged around the light output structure 270.

FIG. 3 is a block view illustrating a sensor circuit 300 of an electronic device 201 according to an embodiment of the disclosure.

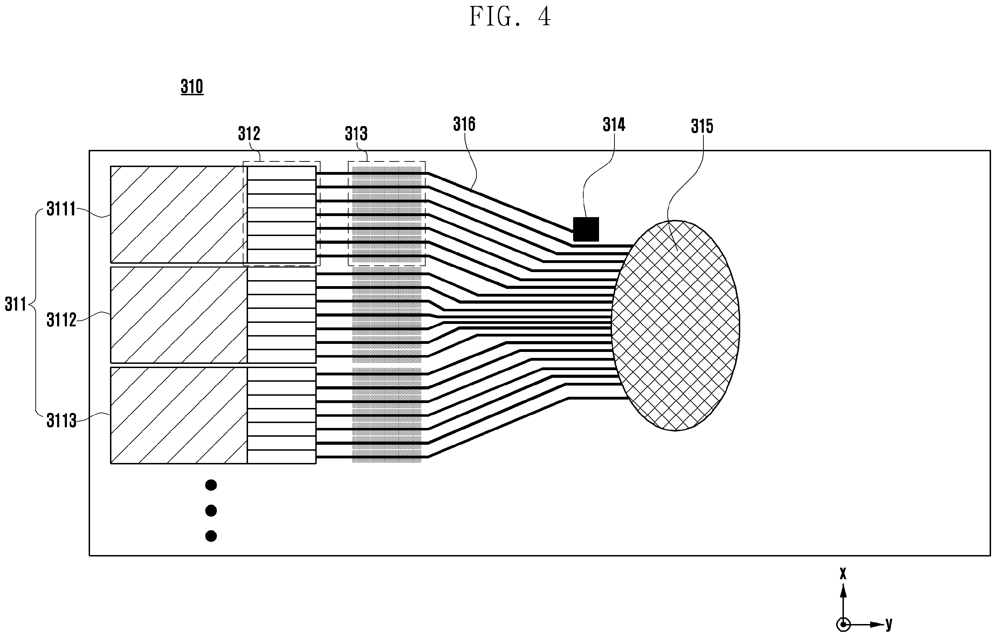

FIG. 4 is a view illustrating a transmission circuit 310 realized on a silicon-based integrated circuit.

Referring to FIGS. 3 and 4, the sensor circuit 300 in FIG. 3 may be identical to the sensor module 176 in FIG. 1 or may include the sensor module 176 of FIG. 1.

In an embodiment, the sensor circuit 300 may include a transmission circuit 310, a processor 320, and/or a reception circuit 330.

In an embodiment, the transmission circuit 310 may include a semiconductor optical amplifier 311, a fixed array distributed Bragg reflector (DBR) grating 312, a modulator 313, a monitoring circuit 314, and/or an output coupler 315.

In an embodiment, the semiconductor optical amplifier 311 may include multiple optical amplifier chips 3111, 3112, and 3113. Without limitation thereto, the semiconductor optical amplifier 311 may include at least one optical amplifier chip.

In an embodiment, the semiconductor optical amplifier 311 may output and/or generate, under control of the processor 320, a broadband laser light. The semiconductor optical amplifier 311 may output and/or generate a laser light with a wavelength of about 2000 nm to about 2400 nm (or about 1500 nm to about 1850 nm). The wavelength of about 2000 nm to about 2400 nm (or about 1500 nm to about 1850 nm) of the laser light may be appropriate to detect blood sugar (or glucose). The multiple optical amplifier chips may output and/or generate, under control of the processor 320, a broadband laser light. The multiple optical amplifier chips may output and/or generate a laser light with a wavelength of about 2000 nm to about 2400 nm (or about 1500 nm to about 1850 nm).

In an embodiment, under control of the processor 320, the semiconductor optical amplifier 311 may control a current and/or a voltage to control output intensity of laser lights output from the respective multiple optical amplifier chips. The electronic device 201 may store a configuration value or configuration data for output intensity of laser lights in the memory 130. Based on the configuration value or configuration data, the electronic device 201 may control a current and/or a voltage to control output intensity of laser lights output from the respective multiple optical amplifier chips.

In an embodiment, blood sugar may absorb at least a portion of multiple modulated laser lights output from the sensor circuit 300. The electronic device 201 may receive multiple modulated laser lights having been transmitted or reflected without being absorbed by blood sugar using a photo detector of the reception circuit 330 and identify a concentration of blood sugar under control of the processor 320 according to an amount (or intensity) and/or wavelength of the received light.

In an embodiment, the semiconductor optical amplifier 311 may output and/or generate, under control of the processor 320, multiple broadband laser lights.

In an embodiment, the semiconductor optical amplifier 311 may output and/or generate, under control of the processor 320, multiple broadband laser lights in time division.

In an embodiment, the multiple optical amplifier chips may output and/or generate, under control of the processor 320, a broadband laser light in time division.

In an embodiment, the processor 320 in FIG. 3 may be identical or similar to the processor 120 in FIG. 1, and the description above is equally applicable to the processor 120 and the processor 320. However, without limitation thereto, the processor 320 in FIG. 3 may be independent from the processor 120 in FIG. 1 and may be included in the sensor circuit 300. The processor 320 may be realized in a form of application-specific integrated circuit (ASIC).

In an embodiment, the fixed array DBR grating 312 may output a laser light having a designated wavelength by modifying a wavelength of the broadband laser light generated and/or output from the semiconductor optical amplifier 311. The designated wavelength may be shorter than a wavelength output from the semiconductor optical amplifier 311.

In an embodiment, the fixed array DBR grating 312 may output multiple laser lights having a designated wavelength by modifying a wavelength of broadband laser lights generated and/or output from multiple semiconductor optical amplifiers 311.

In an embodiment, in response to each optical amplifier chip, the fixed array DBR grating 312 may output multiple laser lights having a designated wavelength by modifying a wavelength of broadband laser lights generated and/or output from each optical amplifier chip.

In an embodiment, the modulator 313 may modulate, under control of the processor 320, multiple laser lights having a designated wavelength output from the fixed array DBR grating 312. In order to improve a signal-to-noise ratio (SNR) of a laser light output, the sensor circuit 310 may modulate multiple laser lights having a designated wavelength output from the fixed array DBR grating 312 using the modulator 313.

In an embodiment, the modulator 313 may modulate a laser light having a designated wavelength into a continuous wave and/or pulse wave.

In an embodiment, the modulator 313 may include a lock-in amplifier and/or a low-pass filter (LPF).

In an embodiment, the modulator 313 may modulate multiple laser lights having a designated wavelength output from the fixed array DBR grating 312 to output modulated multiple laser lights.

In an embodiment, the modulator 313 may transfer the modulated multiple laser lights output to the output coupler 315 through a waveguide (e.g., a waveguide 316 in FIG. 4).

In an embodiment, the output coupler 315 may change an output direction of the modulated multiple laser lights to be output to the outside of the reception circuit 310. For example, referring to FIG. 4, in the transmission circuit 310 realized in a silicon-based integrated circuit, when light travels on the x-y coordinate plane and reaches the output coupler 315, the light may be output along the z-axis, which is perpendicular to the coordinate plane.

In an embodiment, the output coupler 315 may adjust, under control of the processor 320, an output direction and/or angle of the modulated laser lights to be output to the outside of the transmission circuit 310.

In an embodiment, the monitoring circuit 314 may include, for example, and without limitation, an edge illuminated photodiode, a Mach-Zehnder interferometer (MZI) sensor, a ring resonator, a line coupling, and/or a splitter, or the like.

In an embodiment, the monitoring circuit 314 may identify and/or monitor whether modulated multiple laser lights are transferred through a waveguide (e.g., the waveguide 316 in FIG. 4) according to a designated intensity (or power) and a designated wavelength.

In an embodiment, the monitoring circuit 314 may identify at least some laser lights among the modulated multiple laser lights transferred through a waveguide (e.g., the waveguide 316 in FIG. 4).

For example, in case that the light output from the transmission circuit 310 is output deviating from the designated intensity and wavelength, the concentration of blood sugar detected by the processor 320 and/or the electronic device 201 may be measured inaccurately. The monitoring circuit 314 may identify whether modulated multiple laser lights are transferred through a waveguide (e.g., the waveguide 316 in FIG. 4) according to a designated intensity (or power) and a designated wavelength and transfer the identified information to the processor 320. The processor 320 may control the intensity and wavelength of the light output from the semiconductor optical amplifier 311, based on the information received from the monitoring circuit 314. The information may include data related to whether the modulated multiple laser lights are output according to the designated intensity (or power) and designated wavelength.

In an embodiment, the edge illuminated photodiode of the monitoring circuit 314 may detect the intensity of light reaching the monitoring circuit 314. In an embodiment, the Mach-Zehnder interferometer sensor of the monitoring circuit 314 may detect the wavelength of light reaching the monitoring circuit 314.

In an embodiment, the monitoring circuit 314 may be connected between the modulator 313 and the output coupler 315. However, without limitation thereto, the monitoring circuit 314 may be connected between the fixed array DBR grating 312 and the modulator 313.

In an embodiment, the reception circuit 330 may include a photo detector. The reception circuit 330 may include multiple photo detectors. The reception circuit 330 may include at least one photo detector. The photo detector may be spaced a designated distance away from a structure for outputting light in the reception circuit 310. The structure for outputting light in the reception circuit 310 may correspond to the output coupler 315. However, without limitation thereto, the structure for outputting light in the transmission circuit 310 may include a light output structure and/or opening corresponding to the output coupler 315. The photo detector may detect a wavelength in a range of about 2000 nm to about 2400 nm.

In an embodiment, the distance by which the photo detector is spaced apart from a structure (e.g., the output coupler 315) for outputting light in the reception circuit 310 may be various. Multiple distances may be available for the distance by which the photo detector is spaced apart from a structure (e.g., the output coupler 315) for outputting light in the transmission circuit 310. For example, the distance between multiple photo detectors and the structure (e.g., the output coupler 315) for outputting light in the transmission circuit 310 may have multiple designated distance, such as a second distance or a third distance in addition to a first distance.

In an embodiment, the reception circuit 330 may receive light output from the transmission circuit 310. The reception circuit 330 may sequentially receive light output from the transmission circuit 310. The transmission circuit 330 may irradiate multiple modulated laser lights having designated wavelength onto the user's skin in a time division and/or sequential manner. The light irradiated onto the user's skin in a time division and/or sequential manner may penetrate the user's skin and may be received by the reception circuit 330 in a time division and/or sequential manner. Without limitation thereto, the light irradiated onto the user's skin in a time division and/or sequential manner may be reflected from the user's skin and may be received by the reception circuit 330 in a time division and/or sequential manner.

The processor 320 according to an embodiment may include various circuitry (see, e.g., detailed description of processor 120 above) and control the transmission circuit 310 and/or the reception circuit 330. The processor 320 may control the multiple optical amplifier chips (e.g., 3111, 3112, and 3113) included in the semiconductor optical amplifier 311 to output a laser light having a broadband in time division. The processor 320 may control the transmission circuit 310 to output modulated multiple laser lights in time division manner. The processor 320 may control time synchronization of the reception circuit 330 so that the reception circuit 330 may receive the modulated multiple laser lights in a time division manner and/or sequential manner. The processor 320 may demodulate the modulated multiple laser lights received through the reception circuit 330. The processor 320 may include an amplifier and/or a filter to demodulate the modulated multiple laser lights received through the reception circuit 330. However, without limitation thereto, the reception circuit 330 may include an amplifier and/or a filter to demodulate the modulated multiple laser lights. The processor 320 may digitize a photocurrent. The processor 320 may include an analog to digital converter (ADC) for digitizing a photocurrent. However, without limitation thereto, the reception circuit 330 may include an ADC for digitizing a photocurrent. The processor 320 may include a memory (e.g., the memory 130 in FIG. 1) and/or an interface for communication with the processor 120 in FIG. 1.

Referring to FIGS. 3 and 4, the semiconductor optical amplifier 311 may include multiple optical amplifier chips 3111, 3112, and 3113. Although, three optical amplifier chips 3111, 3112, and 3113 are shown in FIG. 4, the semiconductor optical amplifier 311 may include four or more optical amplifier chips.

In an embodiment, each of the multiple optical amplifier chips may output a laser in a designated wavelength band and designated power.

For example, the semiconductor optical amplifier 311 may include at least five optical amplifier chips. In this case, a first optical amplifier chip may output a laser at a first designated wavelength band (e.g., about 2040 to about 2090 nm) and first designated power (e.g., about 5 mW). A second optical amplifier chip may output a laser at a second designated wavelength band (e.g., about 2090 to about 2130 nm) and second designated power (e.g., about 3 to about 5 mW). A third optical amplifier chip may output a laser at a third designated wavelength band (e.g., about 2130 to about 2280 nm) and third designated power (e.g., about 1 to about 3 mW). A fourth optical amplifier chip may output a laser at a fourth designated wavelength band (e.g., about 2280 to about 2330 nm) and fourth designated power (e.g., about 3 to about 5 mW). A fifth optical amplifier chip may output a laser at a fifth designated wavelength band (e.g., about 2330 to about 2380 nm) and fifth designated power (e.g., about 5 mW).

For example, the semiconductor optical amplifier 311 may include at least four optical amplifier chips. In this case, a sixth optical amplifier chip may output a laser at a sixth designated wavelength band (e.g., about 2040 to about 2090 nm) and sixth designated power (e.g., about 5 mW). A seventh optical amplifier chip may output a laser at a seventh designated wavelength band (e.g., about 2090 to about 2210 nm) and seventh designated power (e.g., about 1 to about 3 mW). An eighth optical amplifier chip may output a laser at an eighth designated wavelength band (e.g., about 2310 to about 2330 nm) and eighth designated power (e.g., about 1 to about 3 mW). A ninth optical amplifier chip may output a laser at a ninth designated wavelength band (e.g., about 2330 to about 2380 nm) and ninth designated power (e.g., about 5 mW).

In an embodiment, the semiconductor optical amplifier 311 may divide the entire wavelength band output so as to output lasers with different power according to the designated wavelength band.

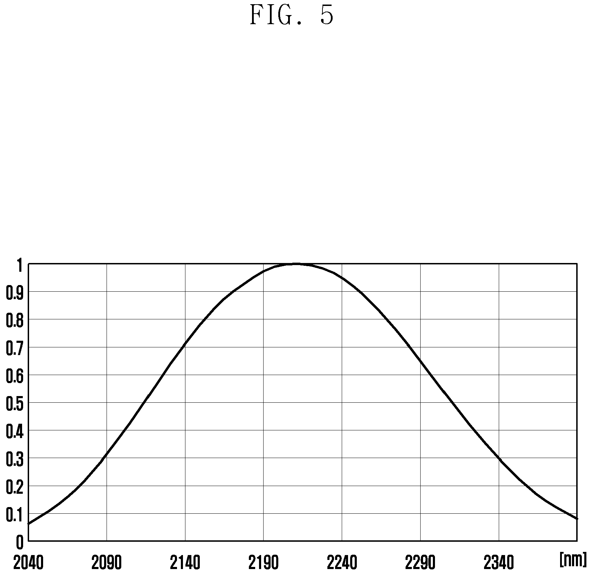

FIG. 5 is a graph depicting density of light according to wavelength after each of multiple optical amplifier chips included in a semiconductor optical amplifier 311 irradiates water with a laser output at an identical level of power.

Referring to FIG. 5, in case that each of the multiple optical amplifier chips outputs a laser at the same level of power, for example, in a wavelength range of about 2130 nm to about 2280 nm, even if a laser is output to water, the power of the laser is not attenuated because water does not absorb light relatively well. In a wavelength range of about 2040 nm to about 2090 nm or about 2330 nm to about 2380 nm, water may absorb light relatively well and thus the power of the laser may be attenuated.

In FIG. 5, the x-axis may represent a wavelength band, and the y-axis may represent a normalized light intensity after light passes through water. FIG. 5 may show attenuation by water depending on the wavelength band.

The semiconductor optical amplifier 311 according to an embodiment of the disclosure may output lasers (e.g., laser light) with different power(s) according to the designated wavelength band by using the multiple optical amplifier chips.

For example, an optical amplifier chip, which outputs a wavelength band of about 2040 to about 2090 nm or about 2330 nm to about 2380 nm, where the laser power attenuation by water is relatively large, may output a laser at a higher level of power than a determined level. An optical amplifier chip, which outputs a wavelength band of about 2130 nm to about 2280 nm, where the laser power attenuation by water is relatively small, may output a laser at a lower level of power than a determined level.

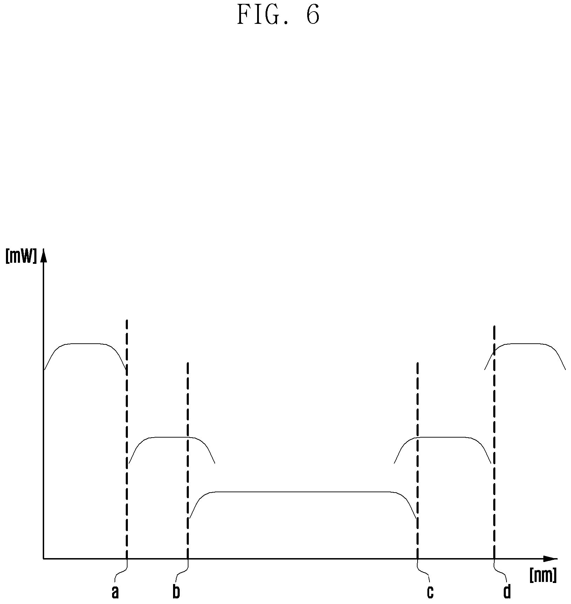

FIG. 6 is a graph illustrating laser power for each wavelength band output from a semiconductor optical amplifier 311 including at least five optical amplifier chips according to an embodiment of the disclosure.

In FIG. 6, the x-axis may represent a wavelength, and the y-axis may represent a light intensity (unit: mW). FIG. 6 may represent a light intensity for each wavelength.

In an embodiment, the semiconductor optical amplifier 311 may output a laser with at least one designated wavelength band and at least one designated power.

In an embodiment, each of the multiple optical amplifier chips may output a laser in a designated wavelength band and designated power.

In FIG. 6, the semiconductor optical amplifier 311 may include at least five optical amplifier chips.

In an embodiment, the semiconductor optical amplifier 311 may divide the entire band by the number of the multiple optical amplifier chips and output lasers having different power for each divided band. For example, the semiconductor optical amplifier 311 may output a laser in a wavelength range of about 2000 nm to about 2400 nm. The semiconductor optical amplifier 311 including five optical amplifier chips may divide the wavelength range of about 2000 nm to about 2400 nm into five.

In an embodiment, the semiconductor optical amplifier 311 may output a laser with power of a lower level than a designated level in a wavelength band in which power attenuation due to light absorption by water is less than a designated attenuation value and output a laser with power of a higher level than a designated level in a wavelength in which power attenuation due to light absorption by water is higher than a designated attenuation value.

In an embodiment, the semiconductor optical amplifier 311 may output multiple lasers having a designated wavelength in a simultaneous manner or in a time-division manner.

In an embodiment, the multiple optical amplifier chips included in the semiconductor optical amplifier 311 may output multiple lasers having a designated wavelength in a simultaneous manner or in a time-division manner.

In an embodiment, a first optical amplifier chip may output a laser at a first designated wavelength band (about 2040 nm to about 'a' nm) (e.g., about 2040 to about 2090 nm) and first designated power (e.g., about 5 mW). A second optical amplifier chip may output a laser at a second designated wavelength band (about 'a' nm to about 'b' nm) (e.g., about 2090 to about 2130 nm) and second designated power (e.g., about 3 to about 5 mW). A third optical amplifier chip may output a laser at a third designated wavelength band (about 'b' nm to about 'c' nm) (e.g., about 2130 to about 2280 nm) and third designated power (e.g., about 1 to about 3 mW). A fourth optical amplifier chip may output a laser at a fourth designated wavelength band (about 'c' nm to about 'd' nm) (e.g., about 2280 to about 2330 nm) and fourth designated power (e.g., about 3 to about 5 mW). A fifth optical amplifier chip may output a laser at a fifth designated wavelength band (about 'd' nm to about 2380 nm) (e.g., about 2330 to about 2380 nm) and fifth designated power (e.g., about 5 mW).

In an embodiment, the semiconductor optical amplifier 311 may control one of the multiple optical amplifier chips and output a laser at the first designated wavelength band (about 2040 nm to about 'a' nm) (e.g., about 2040 to about 2090 nm) and first designated power (e.g., about 5 mW). The semiconductor optical amplifier 311 may control one of the multiple optical amplifier chips and output a laser at the second designated wavelength band (about 'a' nm to about 'b' nm) (e.g., about 2090 to about 2130 nm) and second designated power (e.g., about 3 to about 5 mW). The semiconductor optical amplifier 311 may control one of the multiple optical amplifier chips and output a laser at the third designated wavelength band (about 'b' nm to about 'c' nm) (e.g., about 2130 to about 2280 nm) and third designated power (e.g., about 1 to about 3 mW). The semiconductor optical amplifier 311 may control one of the multiple optical amplifier chips and output a laser at the fourth designated wavelength band (about 'c' nm to about 'd' nm) (e.g., about 2280 to about 2330 nm) and fourth designated power (e.g., about 3 to about 5 mW). The semiconductor optical amplifier 311 may control one of the multiple optical amplifier chips and output a laser at the fifth designated wavelength band (about 'd' nm to about 2380 nm) (e.g., about 2330 to about 2380 nm) and fifth designated power (e.g., about 5 mW).

In an embodiment, the semiconductor optical amplifier 311 may output a laser at the first designated wavelength band (about 2040 nm to about 'a' nm) (e.g., about 2040 to about 2090 nm) and first designated power (e.g., about 5 mW). The semiconductor optical amplifier 311 may output a laser at the second designated wavelength band (about 'a' nm to about 'b' nm) (e.g., about 2090 to about 2130 nm) and second designated power (e.g., about 3 to about 5 mW). The semiconductor optical amplifier 311 may output a laser at the third designated wavelength band (about 'b' nm to about 'c' nm) (e.g., about 2130 to about 2280 nm) and third designated power (e.g., about 1 to about 3 mW). The semiconductor optical amplifier 311 may output a laser at the fourth designated wavelength band (about 'c' nm to about 'd' nm) (e.g., about 2280 to about 2330 nm) and fourth designated power (e.g., about 3 to about 5 mW). The semiconductor optical amplifier 311 may output a laser at the fifth designated wavelength band (about 'd' nm to about 2380 nm) (e.g., about 2330 to about 2380 nm) and fifth designated power (e.g., about 5 mW).

In an embodiment, the semiconductor optical amplifier 311 may control, under control of the processor 320, one of the multiple optical amplifier chips to output a laser at the first designated wavelength band (about 2040 nm to about 'a' nm) (e.g., about 2040 to about 2090 nm) and first designated power (e.g., about 5 mW). The semiconductor optical amplifier 311 may control, under control of the processor 320, one of the multiple optical amplifier chips to output a laser at the second designated wavelength band (about 'a' nm to about 'b' nm) (e.g., about 2090 to about 2130 nm) and second designated power (e.g., about 3 to about 5 mW). The semiconductor optical amplifier 311 may control, under control of the processor320, one of the multiple optical amplifier chips to output a laser at the third designated wavelength band (about 'b' nm to about 'c' nm) (e.g., about 2130 to about 2280 nm) and third designated power (e.g., about 1 to about 3 mW). The semiconductor optical amplifier 311 may control, under control of the processor320, one of the multiple optical amplifier chips and output a laser at the fourth designated wavelength band (about 'c' nm to about 'd' nm) (e.g., about 2280 to about 2330 nm) and fourth designated power (e.g., about 3 to about 5 mW). The semiconductor optical amplifier 311 may control one of the multiple optical amplifier chips to output a laser at the fifth designated wavelength band (about 'd' nm to about 2380 nm) (e.g., about 2330 to about 2380 nm) and fifth designated power (e.g., about 5 mW).

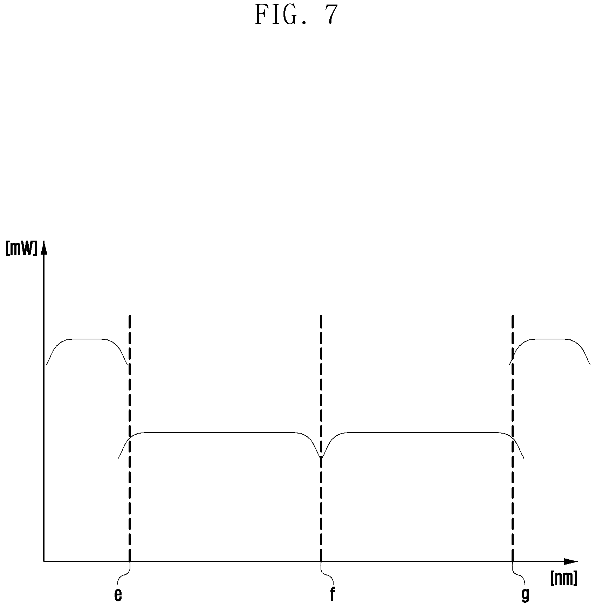

FIG. 7 is a graph illustrating laser power for each wavelength band output from a semiconductor optical amplifier 311 including at least four optical amplifier chips according to an embodiment of the disclosure.

In FIG. 7, the x-axis may represent a wavelength, and the y-axis may represent a light intensity (unit: mW). FIG. 7 may represent a light intensity for each wavelength of the disclosure.

In an embodiment, the semiconductor optical amplifier 311 may divide the entire band by the number of the multiple optical amplifier chips and output lasers having different power for each divided band. For example, the semiconductor optical amplifier 311 may output a laser in a wavelength range of about 2000 nm to about 2400 nm. The semiconductor optical amplifier 311 including four optical amplifier chips may divide the wavelength range of about 2000 nm to about 2400 nm into four.

In an embodiment, the semiconductor optical amplifier 311 may output a laser with power of a lower level than a designated level in a wavelength band in which power attenuation due to light absorption by water is less than a designated attenuation value and output a laser with power of a higher level than a designated level in a wavelength in which power attenuation due to light absorption by water is higher than a designated attenuation value.

In an embodiment, the semiconductor optical amplifier 311 may output a laser with at least one designated wavelength band and at least one designated power.

In an embodiment, each of the multiple optical amplifier chips may output a laser in a designated wavelength band and designated power.

In FIG. 7, the semiconductor optical amplifier 311 may include at least four optical amplifier chips.

In an embodiment, the semiconductor optical amplifier 311 may output multiple lasers having a designated wavelength in a simultaneous manner or in a time-division manner.

In an embodiment, the multiple optical amplifier chips included in the semiconductor optical amplifier 311 may output multiple lasers having a designated wavelength in a simultaneous manner or in a time-division manner.

In an embodiment, a sixth optical amplifier chip may output a laser at a sixth designated wavelength band (about 2040 nm to about 'e' nm) (e.g., about 2040 to about 2090 nm) and sixth designated power (e.g., about 5 mW). A seventh optical amplifier chip may output a laser at a seventh designated wavelength band (about 'e' nm to about 'f' nm) (e.g., about 2090 to about 2210 nm) and seventh designated power (e.g., about 1 to about 3 mW). An eighth optical amplifier chip may output a laser at an eighth designated wavelength band (about 'f' nm to about 'g' nm) (e.g., about 2210 to about 2330 nm) and eighth designated power (e.g., about 1 to about 3 mW). A ninth optical amplifier chip may output a laser at a ninth designated wavelength band (about 'g' nm to about 2380 nm) (e.g., about 2330 to about 2380 nm) and ninth designated power (e.g., about 5 mW).

In an embodiment, the semiconductor optical amplifier 311 may control one of the multiple optical amplifier chips and output a laser at the sixth designated wavelength band (about 2040 nm to about 'e' nm) (e.g., about 2040 to about 2090 nm) and sixth designated power (e.g., about 5 mW). The semiconductor optical amplifier 311 may control one of the multiple optical amplifier chips and output a laser at the seventh designated wavelength band (about 'e' nm to about 'f' nm) (e.g., about 2090 to about 2210 nm) and seventh designated power (e.g., about 1 to about 3 mW). The semiconductor optical amplifier 311 may control one of the multiple optical amplifier chips and output a laser at the eighth designated wavelength band (about 'f' nm to about 'g' nm) (e.g., about 2210 to about 2330 nm) and eighth designated power (e.g., about 1 to about 3 mW). The semiconductor optical amplifier 311 may control one of the multiple optical amplifier chips and output a laser at the ninth designated wavelength band (about 'g' nm to about 2080 nm) (e.g., about 2330 to about 2380 nm) and ninth designated power (e.g., about 5 mW).

In an embodiment, the semiconductor optical amplifier 311 may output a laser at the sixth designated wavelength band (about 2040 nm to about 'e' nm) (e.g., about 2040 to about 2090 nm) and sixth designated power (e.g., about 5 mW). The semiconductor optical amplifier 311 may output a laser at the seventh designated wavelength band (about 'e' nm to about 'f' nm) (e.g., about 2090 to about 2210 nm) and seventh designated power (e.g., about 1 to about 3 mW). The semiconductor optical amplifier 311 may output a laser at the eighth designated wavelength band (about 'f' nm to about 'g' nm) (e.g., about 2210 to about 2330 nm) and eighth designated power (e.g., about 1 to about 3 mW). The semiconductor optical amplifier 311 may output a laser at the ninth designated wavelength band (about 'g' nm to about 2030 nm) (e.g., about 2330 to about 2380 nm) and ninth designated power (e.g., about 5 mW).

In an embodiment, the semiconductor optical amplifier 311 may control, under control of the processor 320, one of the multiple optical amplifier chips to output a laser at the sixth designated wavelength band (about 2040 nm to about 'e' nm) (e.g., about 2040 to about 2090 nm) and sixth designated power (e.g., about 5 mW). The semiconductor optical amplifier 311 may control, under control of the processor 320, one of the multiple optical amplifier chips to output a laser at the seventh designated wavelength band (about 'e' nm to about 'f' nm) (e.g., about 2090 to about 2210 nm) and seventh designated power (e.g., about 1 to about 3 mW). The semiconductor optical amplifier 311 may control, under control of the processor 320, one of the multiple optical amplifier chips to output a laser at the eighth designated wavelength band (about 'f' nm to about 'g' nm) (e.g., about 2210 to about 2330 nm) and eighth designated power (e.g., about 1 to about 3 mW). The semiconductor optical amplifier 311 may control one of the multiple optical amplifier chips to output a laser at the ninth designated wavelength band (about 'g' nm to about 2080 nm) (e.g., about 2330 to about 2380 nm) and ninth designated power (e.g., about 5 mW).

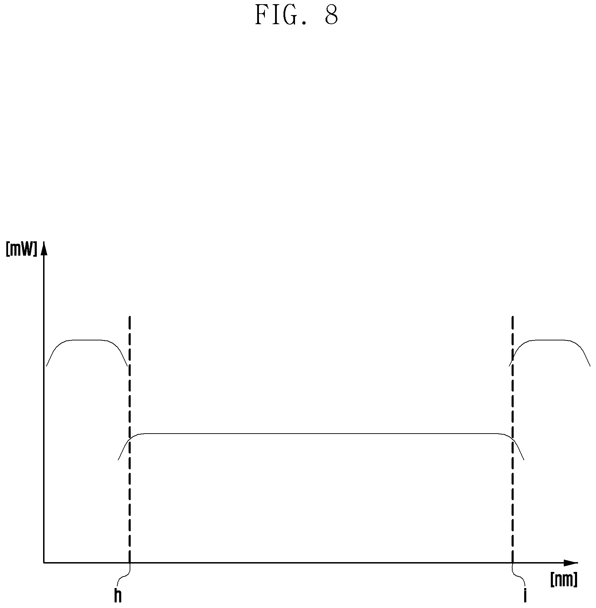

FIG. 8 is a graph illustrating laser power for each wavelength band output from a semiconductor optical amplifier 311 including at least three optical amplifier chips according to an embodiment of the disclosure.

In FIG. 8, the x-axis may represent a wavelength, and the y-axis may represent a light intensity (unit: mW). FIG. 8 may represent a light intensity for each wavelength.

In an embodiment, the semiconductor optical amplifier 311 may divide the entire band by the number of the multiple optical amplifier chips and output lasers having different power for each divided band. For example, the semiconductor optical amplifier 311 may output a laser in a wavelength range of about 2000 nm to about 2400 nm. The semiconductor optical amplifier 311 including three optical amplifier chips may divide the wavelength range of about 2000 nm to about 2400 nm into three.

In an embodiment, the semiconductor optical amplifier 311 may output a laser with power of a lower level than a designated level in a wavelength band in which power attenuation due to light absorption by water is less than a designated attenuation value and output a laser with power of a higher level than a designated level in a wavelength in which power attenuation due to light absorption by water is higher than a designated attenuation value.

In an embodiment, the semiconductor optical amplifier 311 may output a laser with at least one designated wavelength band and at least one designated power.

In an embodiment, each of the multiple optical amplifier chips may output a laser in a designated wavelength band and designated power.

In FIG. 8, the semiconductor optical amplifier 311 may include at least three optical amplifier chips.

In an embodiment, the semiconductor optical amplifier 311 may output multiple lasers having a designated wavelength in a simultaneous manner or in a time-division manner.

In an embodiment, the multiple optical amplifier chips included in the semiconductor optical amplifier 311 may output multiple lasers having a designated wavelength in a simultaneous manner or in a time-division manner.

In an embodiment, a tenth optical amplifier chip may output a laser at a tenth designated wavelength band (about 2040 nm to about 'h' nm) (e.g., about 2040 to about 2090 nm) and tenth designated power (e.g., about 5 mW). An eleventh optical amplifier chip may output a laser at an eleventh designated wavelength band (about 'h' nm to about 'i' nm) (e.g., about 2090 to about 2330 nm) and eleventh designated power (e.g., about 1 to about 3 mW). A twelfth optical amplifier chip may output a laser at a twelfth designated wavelength band (about 'i' nm to about 2380 nm) (e.g., about 2330 to about 2380 nm) and twelfth designated power (e.g., about 5 mW).

In an embodiment, the semiconductor optical amplifier 311 may control one of the multiple optical amplifier chips and output a laser at the tenth designated wavelength band (about 2040 nm to about 'h' nm) (e.g., about 2040 to about 2090 nm) and tenth designated power (e.g., about 5 mW). The semiconductor optical amplifier 311 may control one of the multiple optical amplifier chips and output a laser at the eleventh designated wavelength band (about 'h' nm to about 'i' nm) (e.g., about 2090 to about 2330 nm) and eleventh designated power (e.g., about 1 to about 3 mW). The semiconductor optical amplifier 311 may control one of the multiple optical amplifier chips so that the twelfth optical amplifier chip may output a laser at the twelfth designated wavelength band (about 'i' nm to about 2380 nm) (e.g., about 2330 to about 2380 nm) and twelfth designated power (e.g., about 5 mW).

In an embodiment, the semiconductor optical amplifier 311 may output a laser at the tenth designated wavelength band (about 2040 nm to about 'h' nm) (e.g., about 2040 to about 2090 nm) and tenth designated power (e.g., about 5 mW). The semiconductor optical amplifier 311 may output a laser at the eleventh designated wavelength band (about 'h' nm to about 'i' nm) (e.g., about 2090 to about 2330 nm) and eleventh designated power (e.g., about 1 to about 3 mW). The twelfth optical amplifier chip of the semiconductor optical amplifier 311 may output a laser at the twelfth designated wavelength band (about 'i' nm to about 2380 nm) (e.g., about 2330 to about 2380 nm) and twelfth designated power (e.g., about 5 mW).

In an embodiment, the semiconductor optical amplifier 311 may control, under control of the processor 320, one of the multiple optical amplifier chips to output a laser at the tenth designated wavelength band (about 2040 nm to about 'h' nm) (e.g., about 2040 to about 2090 nm) and tenth designated power (e.g., about 5 mW). The semiconductor optical amplifier 311 may control, under control of the processor 320, one of the multiple optical amplifier chips to output a laser at the eleventh designated wavelength band (about 'h' nm to about 'i' nm) (e.g., about 2090 to about 2330 nm) and eleventh designated power (e.g., about 1 to about 3 mW). The semiconductor optical amplifier 311 may control the twelfth optical amplifier chip to output a laser at the twelfth designated wavelength band (about 'i' nm to about 2380 nm) (e.g., about 2330 to about 2380 nm) and twelfth designated power (e.g., about 5 mW).

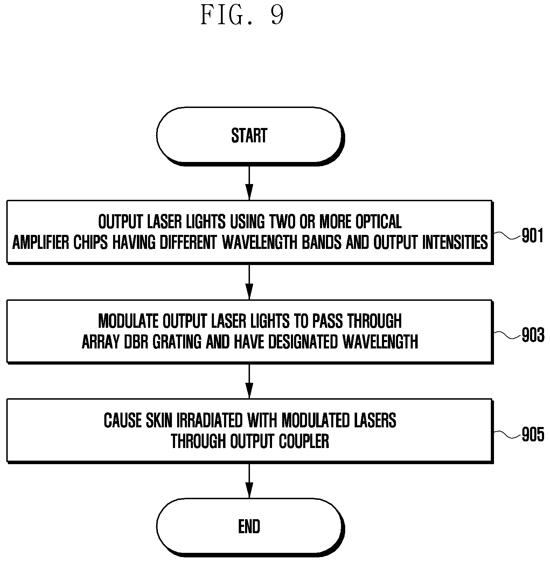

FIG. 9 is a flowchart illustrating a laser output method of an electronic device 201 according to an embodiment of the disclosure.

In an embodiment, in operation 901, the electronic device 201 may cause laser lights having different wavelengths and output intensities to be output by using two or more optical amplifier chips (e.g., 3111, 3112, and 3113).

In an embodiment, in operation 903, the electronic device 201 may modulate the output laser lights to pass through the fixed array DBR grating 312 and have a designated wavelength. The designated wavelength may be shorter than that of laser lights output from the semiconductor optical amplifier 311.

In an embodiment, in operation 905, the electronic device 201 may cause the skin to be irradiated with modulated laser lights through the output coupler 315.

In an embodiment, the electronic device 201 may include a semiconductor optical amplifier 311 configured to divide an outputtable wavelength band to output multiple laser lights having a designated wavelength band and designated power, a fixed array distributed Bragg reflector (DBR) grating 312 configured to modify a wavelength of the multiple laser lights and output multiple laser lights having a modified wavelength, a modulator 313 configured to modulate the multiple laser lights having the modified wavelength, a monitoring circuit 314 configured to identify whether the modulated multiple laser lights are output according to designated power and a designated wavelength, and an output coupler 315 configured to allow the modulated multiple laser lights to adjust an output direction and/or angle and output the modulated multiple laser lights to the outside of the electronic device 201.

In an embodiment, the semiconductor optical amplifier 311 may include multiple optical amplifier chips and output or generate broadband laser lights having a broadband in time division.

In an embodiment, the semiconductor optical amplifier 311 may include a first optical amplifier chip configured to output a laser at a first designated wavelength band and first designated power, a second optical amplifier chip configured to output a laser at a second designated wavelength band and second designated power, a third optical amplifier chip configured to output a laser at a third designated wavelength band and third designated power, and a fourth optical amplifier chip configured to output a laser at a fourth designated wavelength band and fourth designated power.

In an embodiment, the semiconductor optical amplifier 311 may further include a fifth optical amplifier chip configured to output a laser at a fifth designated wavelength band and fifth designated power.

In an embodiment, the semiconductor optical amplifier 311 may output a laser with designated power of a lower level than a designated level in a wavelength band in which power attenuation due to light absorption by water is less than a designated attenuation value.

In an embodiment, the semiconductor optical amplifier 311 may output a laser with designated power of a higher level than a designated level in a wavelength band in which power attenuation due to light absorption by water is higher than a designated attenuation value.

In an embodiment, the semiconductor optical amplifier 311 may divide an outputtable wavelength band by the number of optical amplifier chips included in the semiconductor optical amplifier 311 to be output.

In an embodiment, the modulator 313 may include a lock-in amplifier and/or a low-pass filter.

In an embodiment, the monitoring circuit 314 may include an edge illuminated photodiode, a Mach-Zehnder interferometer (MZI) sensor, a ring resonator, a line coupling, and/or a splitter.

In an embodiment, the monitoring circuit 314 may be connected between the output coupler 315 and the modulator 313.

In an embodiment, the electronic device 201 may further include a reception circuit including multiple photo detectors and each of the multiple photo detectors may be spaced a first designated distance apart from the output coupler 315.

In an embodiment, the electronic device 201 may further include a processor. The processor may control the intensity and wavelength of the light output from the semiconductor optical amplifier 311, based on the information received from the monitoring circuit 314.

In an embodiment, the electronic device 201 may include a transmission circuit, a reception circuit, and a processor, wherein the transmission circuit may include a semiconductor optical amplifier 311 configured to divide an outputtable wavelength band to output multiple laser lights having a designated wavelength band and designated power, a fixed array distributed Bragg reflector (DBR) grating 312 configured to modify a wavelength of the multiple laser lights and output multiple laser lights having a modified wavelength, a modulator 313 configured to modulate the multiple laser lights having the modified wavelength, a monitoring circuit 314 configured to identify whether the modulated multiple laser lights are output according to designated power and a designated wavelength, and an output coupler 315 configured to allow the modulated multiple laser lights to adjust an output direction and/or angle and output the modulated multiple laser lights to the outside of the electronic device 201.

In an embodiment, the semiconductor optical amplifier 311 may include multiple optical amplifier chips and output or generate broadband laser lights having a broadband in time division.

In an embodiment, the semiconductor optical amplifier 311 may output a laser with designated power of a lower level than a designated level in a wavelength band in which power attenuation due to light absorption by water is less than a designated attenuation value.

In an embodiment, the semiconductor optical amplifier 311 may output a laser with designated power of a higher level than a designated level in a wavelength band in which power attenuation due to light absorption by water is higher than a designated attenuation value.

In an embodiment, the semiconductor optical amplifier 311 may divide an outputtable wavelength band by the number of optical amplifier chips included in the semiconductor optical amplifier 311 and output same.

In an embodiment, the electronic device 201 may include a case in which the transmission circuit and the reception circuit are disposed on a rear surface, a display disposed on a front surface of the case, and a band connected to the case and configured to allow the electronic device 201 to be seated on a wrist of the user.

In an embodiment, at least a portion of the rear surface of the case may come in contact with the user through the band when the electronic device 201 is worn on the user.

In an embodiment, at least a portion of the output coupler 315 and at least a portion of the reception circuit may be exposed through the rear surface of the case.

The electronic device according to various embodiments set forth herein may be one of various types of electronic devices. The electronic device may include, for example, a portable communication device (e.g., a smart phone), a computer device, a portable multimedia device, a portable medical device, a camera, a wearable device, or a home appliance. The electronic device according to embodiments of the disclosure is not limited to those described above.

It should be appreciated that the embodiments and the terms used therein are not intended to limit the technological features set forth herein to particular embodiments and the disclosure includes various changes, equivalents, or alternatives for a corresponding embodiment. With regard to the description of the drawings, similar reference numerals may be used to designate similar or relevant elements. It is to be understood that a singular form of a noun corresponding to an item may include one or more of the items, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one or all possible combinations of the items enumerated together in a corresponding one of the phrases. As used herein, such terms as "a first", "a second", "the first", and "the second" may be used to simply distinguish a corresponding element from another, and does not limit the elements in other aspect (e.g., importance or order). If an element (e.g., a first element) is referred to, with or without the term "operatively" or "communicatively", as "coupled with/to" or "connected with/to" another element (e.g., a second element), it means that the element may be coupled/connected with/to the other element directly (e.g., wiredly), wirelessly, or via a third element.

As used in various embodiments of the disclosure, the term "module" may include a unit implemented in hardware, software, or firmware, and may be interchangeably used with other terms, for example, "logic," "logic block," "component," or "circuit". The "module" may be a single integrated component, or a minimum unit or part thereof, adapted to perform one or more functions. For example, according to an embodiment, the "module" may be implemented in the form of an application-specific integrated circuit (ASIC).

Various embodiments as set forth herein may be implemented as software (e.g., the program 140) including one or more instructions that are stored in a storage medium (e.g., the internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it. This allows the machine to be operated to perform at least one function according to the at least one instruction invoked. The one or more instructions each may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, methods according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., Play Store^{™}) or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each element (e.g., a module or a program) of the above-described elements may include a single entity or multiple entities, and some of the multiple entities may be separately disposed in any other element. According to various embodiments, one or more of the above-described elements or operations may be omitted, or one or more other elements or operations may be added. Alternatively or additionally, a plurality of elements (e.g., modules or programs) may be integrated into a single element. In such a case, the integrated component may still perform one or more functions of each of the plurality of components in the same or similar manner as they are performed by a corresponding one of the plurality of components before the integration. According to various embodiments, operations performed by the module, the program, or another element may be carried out sequentially, in parallel, repeatedly, or heuristically, or one or more of the operations may be executed in a different order or omitted, or one or more other operations may be added.

## Claims

1. An electronic device comprising:
a semiconductor optical amplifier configured to divide an outputtable wavelength band and output multiple laser lights having a designated wavelength band and designated power;
a fixed array distributed Bragg reflector (DBR) grating configured to modify a wavelength of multiple laser lights and output multiple laser lights having a modified wavelength; and
an output coupler configured to allow the modulated multiple laser lights to adjust an output direction and/or angle, and output the modulated multiple laser lights to an outside of the electronic device.

2. The electronic device of claim 1, wherein the semiconductor optical amplifier comprises multiple optical amplifier chips and is configured to time-divisionally output or generate the multiple laser lights having a broadband.

3. The electronic device of claim 1, wherein the semiconductor optical amplifier comprises:
a first optical amplifier chip configured to output a laser at a first designated wavelength band and a first designated power;
a second optical amplifier chip configured to output a laser at a second designated wavelength band and a second designated power;
a third optical amplifier chip configured to output a laser at a third designated wavelength band and a third designated power; and
a fourth optical amplifier chip configured to output a laser at a fourth designated wavelength band and a fourth designated power.

4. The electronic device of claim 3, wherein the semiconductor optical amplifier further comprises a fifth optical amplifier chip configured to output a laser at a fifth designated wavelength band and a fifth designated power.

5. The electronic device of claim 1, wherein the semiconductor optical amplifier is configured to output a laser having a designated power of a lower level than a designated level in a wavelength band in which power attenuation due to light absorption by water is less than a designated attenuation value.

6. The electronic device of claim 1, wherein the semiconductor optical amplifier is configured to output a laser having a designated power of a higher level than a designated level in a wavelength band in which power attenuation due to light absorption by water is greater than a designated attenuation value.

7. The electronic device of claim 1, wherein the semiconductor optical amplifier is configured to divide, in outputting the laser lights, an outputtable wavelength band by a number of optical amplifier chips included in the semiconductor optical amplifier.

8. The electronic device of claim 1, further comprising a modulator (313) configured to modulate the multiple laser lights having the modified wavelength,
wherein the modulator comprises a lock-in amplifier and/or a low-pass filter.

9. The electronic device of claim 1, further comprising a monitoring circuit configured to identify whether modulated multiple laser lights are output according to designated power and a designated wavelength,
wherein the monitoring circuit comprises an edge illuminated photodiode, a Mach-Zehnder interferometer (MZI) sensor, a ring resonator, a line coupling, and/or a splitter.

10. The electronic device of claim 9, wherein the monitoring circuit is connected between the output coupler and the modulator.

11. The electronic device of claim 1, further comprising a reception circuit comprising multiple photo detectors,
wherein each of the multiple photo detectors is spaced a first designated distance apart from the output coupler.

12. The electronic device of claim 1, further comprising at least one processor, comprising processing circuitry,
wherein at least one processor, individually and/or collectively, is configured to control an intensity and wavelength of a light output from the semiconductor optical amplifier, based on information received from the monitoring circuit.

13. The electronic device of claim 1, further comprising:
a case in which the transmission circuit and the reception circuit are disposed on a rear surface;
a display disposed on a front surface of the case; and
a band connected to the case and configured to allow the electronic device to be seated on a wrist of the user.

14. The electronic device of claim 13, wherein at least a portion of the rear surface of the case is configured to, based on the electronic device being worn on the user, be in contact with the user through the band.

15. The electronic device of claim 13, wherein at least a portion of the output coupler and at least a portion of the reception circuit are exposed through the rear surface of the case.
